# NEUE EUROPÄISCHE PATENTSCHRIFT

(11) **EP 2 295 039 B2**
(45) Veröffentlichungstag und Bekanntmachung des Hinweises auf die Entscheidung über den Einspruch: **26.10.2022**
(45) Hinweis auf die Patenterteilung: 11.12.2013
(21) Anmeldenummer: 10150491.8
(22) Anmeldetag: 12.01.2010
(51) Int. Cl.: A61K 9/14, A61K 9/50, A61K 38/46, A61K 38/47, A61K 38/48, A61P 1/00, A61P 1/14, A61P 5/48

(54) **Verfahren zur Herstellung von Pankreatin-Pellets, insbesondere Pankreatin-Mikropellets, und hiernach hergestellte Pankreatin-Pellets**
Pancreatin pellets, in particular pancreatin micropellets and method for producing same
Pellets de pancréatine, notamment micro-pellets de pancréatine et leur procédé de fabrication

(30) Priorität: 28.08.2009 DE 202009011699 U
(43) Veröffentlichungstag der Anmeldung: 16.03.2011
(73) Patentinhaber: Nordmark Pharma GmbH, 25436 Uetersen (DE)
(72) Erfinder: Kurfürst, Manfred, Dr., D-25436 Moorrege (DE); Moest, Thomas, Dr., D-25436 Moorrege (DE); Doleschal, Walter, Dr., D-25436 Uetersen (DE)
(74) Vertreter: RGTH

(56) Entgegenhaltungen:
- EP-A1- 0 436 110
- EP-A1- 0 436 110
- TW-B- 310 277
- US-A- 4 280 971
- US-A- 4 280 971
- US-A- 5 378 462
- US-A1- 2007 148 152
- Pancreatin Material Safety Data Sheet (11 November 2005)
- Auszug aus "Engineer's Toolbox' in Bezug auf die Einheit "mesh" (22. August 2014)
- Monography of the European Pharmacopoeia. Pancreas Powder. 2008
- Moisture Analysis - Wikipedia
- Aussagenlogik- Wikipedia
- Auszug der Internetseite "glas-shop.com" zu "Braunen Flaschen"
- Auszug aus "Hunnius Pharmazeutisches Worterbuch", 8. Auflage, 1998
- Auszug der Internetseite "remedia-homoeopathie.de Dosierverschliissen fur Globulin
- Auszug der Internetseite "Globuli.de" zu "Dosierhilfen fur Globuli''

## Beschreibung

Die Erfindung betrifft ein Verfahren zur Herstellung von Pankreatin-Pellets, insbesondere Pankreatin-Mikropellets.

Als Pankreatin bezeichnet man das extrahierte Gemisch der Enzyme der Pankreasdrüse, bestehend im Wesentlichen aus Lipasen, Amylase und Proteasen. Als Ausgangsmaterial für die Herstellung von Pankreatin dient hauptsächlich Schweinepankreas in frischem oder gefrorenem Zustand, dem ursprünglich für die Herstellung von Pankreatin lediglich das Wasser und das Fett entzogen wurden. Im Hinblick auf die Empfindlichkeit der Enzyme sind jedoch Verfahren entwickelt worden, die eine möglichst schonende Gewinnung des Pankreatin ermöglichen sollen. Ein geeignetes Verfahren wird in DE 32 48 588 A1 beschrieben.

Pankreatin wird als Wirkstoff insbesondere zur Behandlung von Verdauungsstörungen, die auf Pankreasinsuffizienz beruhen angewendet. Pankreatin wird hautsächlich in getrockneter Form als orales Therapeutikum verwendet. Dabei hat sich herausgestellt, dass die therapeutische Wirksamkeit der Pankreatingabe verbessert werden kann, wenn der Wirkstoff in Form von Pellets oder Mikropellets verabreicht wird.

Typischerweise werden Pankreatin-Pellets hergestellt, indem dem Pankreatin mit Hilfsstoffen und Bindemitteln versetzt und diese Komponenten vermischt werden, bis ein homogenes Gemisch erhalten wird. Das homogene Gemisch wird in einen Extruder eingebracht, wo das Gemisch in ein strangförmiges Extrudat überführt wird. Das Extrudat wird schließlich in einen Sepheronizer, gegebenenfalls unter Zusatz weiterer Hilfsstoffe eingebracht, in dem die Stränge unter Erhalt von kugelförmigen Pellets zerstört werden. Die Pellets werden anschließend getrocknet und gesiebt, wobei Siebfraktionen mit Pellets, die einen vorgegebenen Größenbereich über- oder untersteigen, ausgesondert werden. Die so erhaltenen Pellets, die eine enge Größenverteilung aufweisen, können anschließend mit einem Überzug aus einem magensaftresistenten Material lackiert werden.

Ein derartiges Verfahren zur Herstellung von Pankreatin-Mikropellets wird beispielsweise in EP 0 583 726 A2 beschrieben. Gemäß diesem Verfahren werden vor der Extrusion 100 Gewichtsteile Pankreatin mit 15 bis 50 Gewichtsteilen Polyethylenglykol 4000 und 10 bis 30 Gewichtsteilen eines Alkohols vermischt. Der Alkohol, beispielweise Propanol, soll dem Gemisch in eine extrudierbare Form verleihen. Die mittels der Extrusion erhaltenen Stränge werden vor der Überführung in den Sepheronizer mit 1,5 bis 5 Gewichtsteilen Paraffin und weiteren 1,5 bis 10 Gewichtsteilen Alkohol versetzt. Die erhaltenen Pellets besitzen einen Pankreatingehalt von 65 bis 85 Gew.-%, so dass die Pellets wenigstens 15 Gew.-% Hilfsstoffe und Bindemittel enthalten.

Die zur Extrusion erforderlichen Hilfsstoffe und Bindemittel können jedoch unerwünschte Nebenwirkungen haben. Aus diesem Grunde wird die Auswahl der Hilfsstoffe und Bindemitte einer ständigen Überprüfung unterzogen. Beispielsweise hat sich herausgestellt, dass der bislang übliche Zusatz mineralischer Öle nicht mehr als unkritisch angesehen werden kann.

Aus diesem Grunde wurde in EP 1 931 317 B1 ein Verfahren vorgeschlagen, mit dem es möglich sein soll, Pankreatin-Pellets ohne den Zusatz von Paraffin herzustellen. Die dort erhaltenen Pankreatin-Pellets enthalten 10 bis 95 Gew.-% Pankreatin, wobei zumindest 5 Gew.-% Hilfsstoffe und Bindemittel wie Polyethylenglycol sind.

Es ist jedoch zu erwarten, dass gegen Zusätze jeder Art begründete oder auch unbegründete Bedenken erhoben werden. Es ist daher wünschenswert, ein Pankreatin in einer Form bereitzustellen, die zum einen oral verabreicht werden kann und zum anderen so weitgehend wie möglich, frei von irgendwelchen Zusätzen ist.

Die US 4,280,971 A offenbart ein Verfahren zur Herstellung von Pankreatinpellets.

Nach der EP 0 436 110 A ist ein Verfahren zur Herstellung von kugelförmigen Pankreatinteilchen bekannt.

Nach der US 5,378,462 A sind mit einem magensaftresistenten Film überziehbare Pankreatinmikropelletkerne bekannt.

Aufgabe der Erfindung ist es, die Nachteile nach dem Stand der Technik zu beseitigen. Es soll insbesondere ein Verfahren zur Herstellung von Pankreatin-Pellets bereitgestellt werden, die über verbesserte Eigenschaften verfügen und eine hohe Wirkstoffkonzentration aufweisen, wobei die Pellets zur Vermeidung einer Beeinträchtigung der pharmakologischen Wirkung durch zugesetzte Hilfsstoffe oder Bindemittel das Pankreatin unter Ausschluss von Zusatzstoffen und Bindemitteln und anderweitigen Hilfsstoffen in möglichst aktiver Form enthalten sollen und deren orale Verabreichung der Pellets erleichtert werden soll. Diese Aufgabe wird durch die Merkmale des Anspruches 1 gelöst. Zweckmäßige Ausgestaltungen der Erfindung ergeben sich aus den Merkmalen der Unteransprüche.

Nach Maßgabe des erfindungsgemäßen Verfahrens wird ein Pankreatin-Pellet erhalten, das ausschließlich aus Pankreatin gebildet ist. Das durch das erfindungsgemäße Verfahren erhaltene Pankreatin-Pellet besteht somit zu 100 % aus Pankreatin und enthält keine Hilfsstoffe und Bindemittel.

Weitere vorteilhafte Ausgestaltungen der Erfindung sind Gegenstand der Unteransprüche.

In einer Ausführungsform des erfindungsgemäßen Verfahrens ist das Pankreatin-Pellet ein Mikropellet. Unter einem Pellet wird in der vorliegenden Erfindung ein Körper mit einer kugeligen oder ellipsoiden Gestalt verstanden, wobei gemäß der Erfindung der Kugeldurchmesser bzw. die kurze Achse im Bereich von 0,4 bis 0,8 mm liegt, oder mit einer tropfenförmigen Gestalt zur Ausgabe aus einer Tropfenflasche.

Das Pankreatin wurde vorzugsweise aus dem Pankreas eines Säugetieres, vorzugsweise aus dem Pankreas eines Schweins oder eines Rindes gewonnen. Die Herstellung der Pellets ist in das Verfahren zur Gewinnung des Pankreatin integriert. Der Zusatz eines gesonderten Hilfsstoffes zur Herstellung einer plastischen Masse, wie dies nach dem Stand der Technik für eine Pelletierung unter Verwendung eines Extruders erforderlich, ist daher nicht notwendig.

Die Pellets werden somit durch folgende Vorgehensweise erhalten: Die vom Schwein oder Rind stammenden Pankreasdrüsen werden zunächst zerkleinert und einer Autolyse unterzogen. Durch Filtration des so erhaltenen Zwischenproduktes wird das Siebfiltrat gewonnen. Anschließend werden die Enzyme, die sich in dem Siebfiltrat befinden, ausgefällt, das Gemisch filtriert und der Filterkuchen gewonnen. Der Filterkuchen wird schließlich gemahlen und vakuumgetrocknet, bis er eine Restfeuchte, die 0,1 bis 0,3 Gew.-% beträgt, aufweist. Das beschriebene Pankreatinprodukt mit 0,3 % Restfeuchte ist das getrocknete Endprodukt, wohingegen die extrudierbare Filterkuchenmasse mehr Restfeuchte / Organlösungsmittelrückstände enthält, die in der Größenordnung von 50 % liegen. Anschließend wird der Filterkuchen einer Wärmebehandlung bei 80° C oder weniger unterzogen. Der Filterkuchen ist überraschenderweise unmittelbar ohne weitere Zusatzstoffe und Bindemittel extrudierbar und sphäronisierbar. Danach werden die erhaltenen Pellets getrocknet. Der wärmebehandelte Filterkuchen weist eine hinreichende Plastizität auf, um eine Extrusion zur Bildung von Strängen aus dem Filterkuchen erreichen zu können. Nach der Extrusion kann eine Spheronisation vorgenommen werden, und zwar ebenfalls ohne Zusatz von Zusatzstoffen und Bindemitteln bzw. anderweitigen Hilfsstoffen. Dabei werden kugelige, elliptische oder tropfenförmige Pellets erhalten, die wie sie sind oder als Kerne, die mit einem Überzug versehen werden sollen, verwendet werden können.
**f** Der Kern besteht damit zu 100 % aus Pankreatin und enthält keine Hilfsstoffe und Bindemittel. Hingegen kann der Überzug aus zumindest einem Hilfsstoff und/oder zumindest einem Bindemittel gebildet sein.

In einer anderen Ausführungsform besteht der Überzug aus einer ersten, inneren Schicht, die den Kern aus Pankreatin umhüllt, und einer zweiten, äußeren Schicht. Vorzugsweise ist die erste Schicht aus zumindest einem Hilfsstoff und/oder zumindest einem Bindemittel gebildet. Die zweite Schicht ist vorzugsweise aus einem magensaftresistenten Material gebildet.

Der Gewichtsanteil von Hilfsstoffen und Bindemitteln an dem Pankreatin-Pellet sollte zwischen 5 und 30 Gew.-% liegen. Der Gewichtsanteil des magensaftresistenten Materials an dem Pankreatin-Pellet sollte zwischen 10 und 30 Gew.-% liegen.

Die Hilfsstoffe und Bindemittel, die den Kern aus Pankreatin umschließen, bewirken einen Zusammenhalt des Kernes beispielweise während der Lagerung und des Transports der Pellets und verhindern damit eine mechanische oder chemische Zerstörung des Kerns. Die verwendeten Hilfsstoffe und Bindemittel müssen pharmakologisch verträglich sein.

Geeignete pharmakologisch verträgliche Hilfsstoffe, die den Überzug oder die innere Schicht des Überzuges, gegebenenfalls gemeinsam mit den nachstehend beschriebenen Bindemitteln bilden können, umfassen zum Beispiel Füllstoffe, Feuchthaltemittel, Gleitmittel, Desintegrationsmittel und Färbemittel. Diese Aufzählung ist nicht abschließend, vielmehr können andere, dem Fachmann bekannte Hilfsstoffe eingesetzt werden.

Beispiele geeigneter Füllstoffe sind aus der Gruppe ausgewählt, die Calciumphosphat, mikrokristalline Cellulose, Dextrane, Dextrin, gefälltes Calciumcarbonat, hydratisiertes Siliciumdioxid, Kaolin, Lactose, Mannitol, Maisstärke, Polyvinylpyrrolidon, Sorbitol, Talkum und Gemische davon umfasst. Beispiele geeigneter Feuchthaltemittel sind aus der Gruppe ausgewählt, die Glycerol, Stärke und Gemische davon, umfasst. Beispiele geeigneter Desintegrationsmittel sind aus der Gruppe ausgewählt, die, Alginsäure, Amylose, Calciumalginat, Calciumcarbonat, Formaldehydgelatine, Natriumhydrogencarbonat, Kieselsäure, Sagostärke, Stärke und Gemische davon umfasst. Geeignete Gleitmittel, sind aus der Gruppe ausgewählt, die beispielsweise Calcium- oder Magnesiumstearat, Stärke, Stearinsäure, Talkum und Gemische davon umfasst.

Geeignete pharmakologisch verträgliche Bindemittel, die den Überzug oder die innere Schicht des Überzuges, gegebenenfalls gemeinsam mit den vorstehend beschriebene Hilfsstoffen bilden können, sind beispielsweise Verbindungen, die aus der Gruppe ausgewählt sind, die Hydroxypropylmethylcellulose, Polyethylenglycole wie Polyethylenglycol 1500, Polyethylenglycol 2000, Polyethylenglycol 3000, Polyethylenglycol 4000, Polyethylenglycol 6000, Polyethylenglycol 8000, Polyethylenglycol 10000, Polyoxyethylen, Polyoxyethylen-Polyoxypropylen-Copolymere und Gemische davon umfasst. Diese Aufzählung ist nicht abschließend, vielmehr können andere, dem Fachmann bekannte Bindemittel eingesetzt werden. Geeignete Farbstoffe sind beispielweise Lebensmittelfarbstoffe, insbesondere Lebensmittelfarbstoffe, die in der deutschen Arzneimittelfarbstoffverordnung beschrieben sind. Diese Aufzählungen sind nicht abschließend, vielmehr können andere, dem Fachmann bekannte Hilfsstoffe eingesetzt werden.

Die Bildung des Überzuges aus Hilfsstoffen und/oder Bindemitteln bzw. der inneren Schicht des Überzuges kann mittels bekannter Techniken, beispielsweise in einer Wurster-Kolone oder einem Kugelcoater, durchgeführt werden. Für eine Beschichtung im Kugelcoater werden die Pellets, die ausschließlich aus Pankreatin bestehen, in den Kugelcoater eingebracht und ein zuvor hergestelltes homogenes Gemisch aus dem Bindemittel und den Hilfsstoffen eingesprüht.

Der magensaftresistente Überzug verhindert eine Zerstörung des Pankreatins im Magen aufgrund der Einwirkung der Magensäure auf die säurelabilen Bestandteile des Pankreatins, insbesondere der Lipasen. Nach der Magenpassage und der Änderung des pH-Wertes beim Eintritt in den Dünndarm löst sich der Schutzfilm, den der magensaftresistente Überzug um den Kern aus Pankreatin gebildet hat, auf, so dass das Pankreatin freigesetzt wird. Das magensaftresistente Material muss bei einem pH-Wert von bis zu 5,5 stabil sein und erst bei einem pH von 5,5 und höher, vorzugsweise 6 und höher die Freisetzung des Pankreatins zulassen.

Geeignete magensaftresistente Materialien, die zur Bildung von Überzügen von Pellets verwendet werden, sind aus dem Stand der Technik bekannt. Üblicherweise enthalten solche Überzüge einen Filmbildner, zumeist einen Weichmacher und in einigen Fällen ein Trennmittel. Geeignete Filmbildner sind beispielsweise aus der Gruppe ausgewählt, die Hydroxypropylmethylcelluloseacetatsuccinat (HPMCAS), Hydroxypropylmethylcellulosephthalat (HPMCP), Celluloseacetatphthalat (CAP), Polyvinylacetatphthalat (PVAP), MethacrylsäureMethylmethacrylat-Copolymerisate und Methacrylsäure-Ethylacrylat-Copolymerisate sowie Gemische davon umfasst. Diese Aufzählung ist nicht abschließend, vielmehr können andere, dem Fachmann bekannte Filmbildner eingesetzt werden.

Enthält das magensaftresistente Material neben dem Filmbildner einen Weichmacher, so sollte dessen Anteil in Bezug auf den Filmbildner zwischen 1 und 20 Gew.-% liegen. Bevorzugte Weichmacher sind einwertige Alkohole mit 12 bis 30 Kohlenstoffatomen wie beispielsweise Cetylalkohol, Stearylalkohol, Triethylalkohol und Gemische davon. Diese Aufzählung ist nicht abschließend, vielmehr können andere, dem Fachmann bekannte Weichmacher eingesetzt werden.

Enthält das magensaftresistente Material neben dem Filmbildner und gegebenenfalls dem Weichmacher auch ein Trennmittel, so sollte dessen Anteil zwischen 0,5 und 5 Gew.-%, bezogen auf den Filmbildner, liegen. Beispielhafte Trennmittel sind Castoröl und Dimethicon. Diese Aufzählung ist nicht abschließend, vielmehr können andere, dem Fachmann bekannte Trennmittel eingesetzt werden.

Zur Erzeugung des Überzuges aus dem magensaftresistenten Material können der Filmbilder, gegebenenfalls der Weichmacher und/oder das Trennmittel, in bekannter Art und Weise in einem Lösungsmittel gelöst oder dispergiert werden. Das Lösungsmittel wird nach der Bildung des Überzuges, beispielsweise durch Trocknen, entfernt. Geeignete Lösungsmittel sind aus der Gruppe ausgewählt, die Wasser, Aceton, Alkohole mit 1 bis 5 Kohlenstoffatomen wie Methanol, Ethanol, nund isoPropanol, n- und tert-Butanol oder Gemische davon umfasst.

Die Bildung des magensaftresistenten Überzuges kann mittels bekannter Techniken, beispielsweise in einer Wurster-Kolone oder einem Kugelcoater, durchgeführt werden. Für eine Beschichtung im Kugelcoater werden die Pellets, die bereits mit dem Überzug aus den Hilfsstoffen und dem Bindemittel beschicht sind, in den Kugelcoater eingebracht und das magensaftresistente Material eingesprüht.

Vorzugsweise ist der Kern von kugeliger Gestalt, während der Überzug dem Pankretin-Pellet eine tropfenförmige Gestalt verleihen kann, was die Dosierung der Pellets, beispielweise aus Tropfenflaschen, erleichtert.

Eine tropfenförmige Gestaltung bietet den Vorteil, dass sich die Pellets beim Fall in einer Richtung orientieren. Dies erlaubt ein einwandfreies Mitzählen der aus einem Vorratsbehälter, wie einer Tropfenflasche, ausgegebenen tropfenförmigen Pankreatin-Pellets.

Die nachfolgenden Zeichnungen zeigen gemäß des Verfahrens herstellbare Pellets. Dabei zeigen:
- Fig. 1: eine Schnittdarstellung einer ersten Ausführungsform eines Pankreatin-Pellets, das ausschließlich aus Pankreatin gebildet ist;
- Fig. 2: eine Schnittdarstellung einer zweiten Ausführungsform eines Pankreatin-Pellets mit einem Pankreatin-Kern und einem einlagigen Überzug;
- Fig. 3: eine Schnittdarstellung einer dritten Ausführungsform eines Pankreatin-Pellets mit einem Pankreatin-Kern und einem zweilagigen Überzug;
- Fig. 4: eine Schnittdarstellung einer vierten Ausführungsform eines Pankreatin-Pellets mit einem Pankreatin-Kern und einem einlagigen Überzug in Tropfenform; und
- Fig. 5: eine Schnittdarstellung einer fünften Ausführungsform eines Pankreatin-Pellets mit einem Pankreatin-Kern und einem zweilagigen Überzug in Tropfenform.

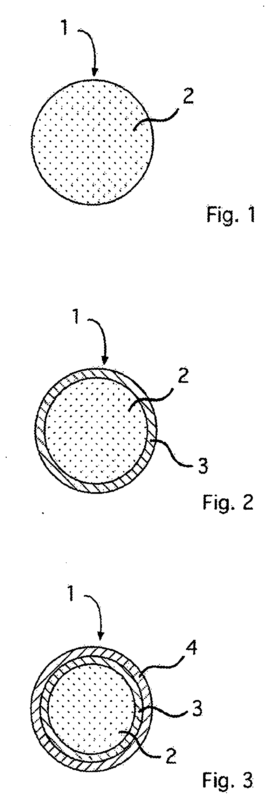

Die in Fig. 1 gezeigte erste Ausführungsform eines Pankreatin-Pellets 1 lässt erkennen, dass dieses Pellet 1 ausschließlich aus Pankreatin 2 gebildet. Es weist keinen Überzug auf.

Im Gegensatz dazu besitzt die in Fig. 2 gezeigte zweite Ausführungsform eines Pankreatin-Pellets 1 einen Kern 2 und einen einlagigen Überzug 3. Der Kern 2 besteht ausschließlich aus Pankreatin. Der Überzug ist aus Hilfsmitteln und Bindemittel gebildet. Der Überzug 3 bewirkt einen Zusammenhalt des Pankreatinkernes 2 und verhindert beispielsweise eine mechanische oder chemische Zerstörung des Kerns 2.

Die in Fig.3 gezeigte dritte Ausführungsform des Pankreatin-Pellets weist einen zweilagigen Überzug auf. Der innere Überzug 3 umhüllt den Kern 2 aus Pankreatin. Auf diese Weise wird der Pankreatin-Kern zusammengehalten und gegen mechanische oder chemische Zerstörung geschützt. Die äußere Lage 4 besteht aus einem magensaftresistenten Material.

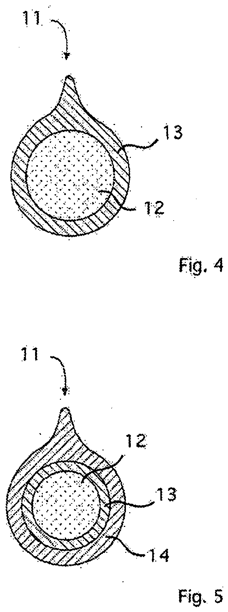

In den Fig. 4 und 5 ist der Überzug tropfenförmig ausgebildet. Bei der in Fig. 4 gezeigten Ausführungsform weist das Pankreatin-Pellet 11 einen einlagigen Überzug 13 in Tropfenform auf, der den kugelförmigen Kern 12, der ausschließlich aus Pankreatin besteht, umhüllt. Der Überzug 13 besteht dabei aus Hilfsstoffen und Bindemitteln. In Fig. 5 ist der Überzug zweilagig und weist eine innere Schicht 13 und eine äußere Schicht 14 auf. Dabei weist nur die Schicht 14 eine tropfenartige Form auf, während die innere Schicht 13 kugelförmig ist. Die innere Schicht 13 besteht aus Hilfsmitteln und Bindemitteln, die den Kern 13 zusammenhalten. Die äußere Schicht 14 besteht aus einem magensaftresistenten Material.

### Bezugszeichenliste

- 1: kugelförmiges Pankreatin-Pellet
- 2: Pankreatin
- 3: Überzug aus Hilfsstoffen und Bindemittel
- 4: Überzug aus einem magensaftresistenten Material
- 11: tropfenförmiges Pankreatin-Pellet
- 12: Pankreatin
- 13: Überzug aus Hilfsstoffen und Bindemittel
- 14: Überzug aus einem magensaftresistenten Material

## Patentansprüche

1. Verfahren zur Herstellung eines zu 100 % aus Pankreatin bestehenden Pellets bzw. Mikropellets ohne Hilfsstoffe und Bindemittel und mit einem Überzug oder ohne einen Überzug sowie mit einer kugeligen oder ellipsoidischen Gestalt, wobei der Kugeldurchmesser bzw. die kurze Achse im Bereich von 0,4 bis 0,8 mm liegt oder mit einer tropfenförmigen Gestalt zur Ausgabe aus einer Tropfenflasche
umfassend folgende Schritte:
a.) Zerkleinerung von vom Schwein oder Rind stammenden Pankreasdüsen unter Unterziehung einer Autolyse;
a1.) Gewinnung eines Siebfiltrats durch Filtration des nach Schritt a.) erhaltenen Zwischenproduktes;
a2.) Ausfällung der Enzyme aus dem Siebfiltrat;
a3.) Filtrierung des nach Schritt a2.) erhaltenen Gemisches zur Gewinnung des Filterkuchens;
a4.) Vermahlung und Vakuumtrocknung des Filterkuchens bis zum Erhalt einer Restfeuchte von 0,1 bis 0,3 Gew.-%, wobei das Pankreatinprodukt mit 0,1 bis 0,3 Gew.-% Restfeuchte das getrocknete Endprodukt bildet, wohingegen die extrudierbare Filterkuchenmasse eine in der Größenordnung von 50% liegende Restfeuchte bzw. Organlösemittelrückstände enthält;
a5.) Unterziehung des Filterkuchens einer Wärmebehandlung bei 80°C oder bei einer unter 80°C liegenden Temperatur;
a6.) Extrudierung des wärmebehandelten und eine hinreichende Plastizität aufweisenden Filterkuchens unter Ausschluss von Zusatzstoffen und Bindemitteln zur Bildung von Strängen;
a7.) Spheronisation unter Ausschluss von Zusatzstoffen und Bindemitteln oder anderweitigen Hilfsstoffen zur Gewinnung von kugeligen, elliptischen oder tropfenförmigen Pellets.

2. Verfahren nach Anspruch 1, **dadurch gekennzeichnet, dass** die Pellets bzw. Mikropellets mit einem Überzug aus einer ersten inneren Schicht aus Hilfsstoffen und Bindemitteln mit einem zwischen 5 und 30 Gew.-% liegenden Gewichtsanteil und einer zweiten äußeren Schicht aus einem magensaftresistenten Material mit einem zwischen 10 und 30 Gew.-% liegenden Gewichtsanteil versehen wird.

## Claims

1. Method for the preparation of a pellet or micropellet consisting of 100% pancreatin without excipients and binders and with a coating or without a coating and having a spherical or ellipsoidal shape, wherein the sphere diameter or short axis, respectively, is in the range of 0.4 to 0.8 mm, or having a drop shape for dispensing from a drop bottle
comprising the following steps:
a.) comminuting pancreatic glands originating from pigs or cattle, while subjecting to autolysis;
a1.) obtaining a sieve filtrate by filtration of the intermediate product obtained after step a.);
a2.) precipitating the enzymes from the sieve filtrate;
a3.) filtrating the mixture obtained after step a2.) to obtain the filter cake;
a4.) grinding and vacuum drying the filter cake until a residual moisture of 0.1 to 0.3% by weight is obtained, wherein the pancreatin product with 0.1 to 0.3% by weight residual moisture forms the dried end product, whereas the extrudable filter cake mass contains a residual moisture of the order of 50% or organ solvent residues, respectively;
a5.) subjecting the filter cake to a heat treatment at 80°C or at a temperature lower than 80°C;
a6.) extruding the heat-treated filter cake, which has sufficient plasticity, with the exclusion of additives and binders to form strands;
a7.) spheronization with exclusion of additives and binders or other auxiliary substances to obtain spherical, elliptical or drop-shaped pellets.

2. Method according to claim 1, **characterized in that** the pellets or micropellets, respectively, are provided with a coating consisting of a first inner layer of excipients and binders with a weight fraction lying between 5 and 30% by weight and a second outer layer consisting of an entericcoated material with a weight fraction lying between 10 and 30% by weight.

## Revendications

1. Procédé de fabrication d'un granulé ou micro-granulé constitué à 100 % de pancréatine, sans agents auxiliaires et liants, pourvu ou dépourvu d'un enrobage, ayant en outre une forme sphérique ou ellipsoïde, le diamètre de la sphère, ou l'axe court, étant compris entre 0,4 mm et 0,8 mm ; ou bien ayant une forme de goutte destinée à être dispensée par un flacon compte-gouttes,
comprenant les étapes suivantes :
a.) broyer des glandes pancréatiques d'origine porcine ou bovine, tout en intégrant la réalisation d'une autolyse ;
a1.) produire un filtrat de tamisage en soumettant le produit intermédiaire obtenu à l'issue de l'étape a.) à une filtration ;
a2) précipiter les enzymes contenues dans le filtrat de tamisage ;
a3.) filtrer le mélange obtenu à l'issue de l'étape a2.) pour ainsi récupérer le culot de filtration ;
a4.) broyer puis sécher sous vide ledit culot de filtration jusqu'à obtenir un taux d'humidité résiduelle compris entre 0,1 et 0,3 % en poids, le produit de pancréatine, dont le taux d'humidité résiduelle est compris entre 0,1 et 0,3 % en poids, constituant le produit final alors que la masse de culot de filtration susceptible d'être extrudée contient une humidité résiduelle dans l'ordre de grandeur de 50 % et/ou des résidus de solvants organiques ;
a5.) soumettre le culot de filtration à un traitement thermique à 80 °C ou à une température inférieure à 80 °C ;
a6.) extruder le culot de filtration, qui suite à son traitement thermique présente une plasticité suffisante, sans ajout d'additifs ni de liants, pour ainsi former des cordons ;
a7.) sphéronisation, sans ajout d'additifs ni de liants ni d'autres agents auxiliaires, pour ainsi produire des granulés sphériques, elliptiques ou en forme de gouttes.

2. Procédé selon la revendication 1, **caractérisé en ce que** les granulés ou micro-granulés sont pourvus d'un enrobage constitué d'une première couche intérieure qui est constituée d'agents auxiliaires et de liants et dont la proportion pondérale est comprise entre 5 et 30 % en poids, et d'une deuxième couche extérieure qui est constituée d'un matériau entérique et dont la proportion pondérale est comprise entre 10 et 30 % en poids.
